# EUROPEAN PATENT APPLICATION

(11) **EP 2 740 483 A1**
(43) Date of publication of application: **11.06.2014**
(21) Application number: 11870527.6
(22) Date of filing: 05.08.2011
(51) Int. Cl.: A61K 36/06, A61P 17/02, C12R 1/825

(54) **METHOD FOR TREATING INFECTIOUS SKIN DISEASES USING LIVE "PENICILLIUM NOTATUM" FUNGUS**

(71) Applicant: Chernov, Konstantin Evstafyevich, Norilsk,Krasnoyarskiy Kray 663318 (RU)
(72) Inventor: CHERNOV, Aleksey Konstantinovich, Norilsk Krasnoyarskiy Kray 663318 (RU)
(74) Representative: Spengler, Robert
(86) International application number: PCT/RU2011/000592
(87) International publication number: WO 2013/022363

(57) **Abstract**

Method for treating skin diseases using live "Penicillium notatum" fungus. There is still little known about the "Penicillium notatum" fungus.

Live "Penicillium notatum" fungus has the following functions in the treatment process:
1) as a biological producer to develop the most effective antibiotic against (particular) pathological microorganisms acting as the causative agents of a particular disease,
2) as a biological therapeutic instrument for the rational delivery of an antibiotic individually to each pathogenic microorganism,
3) as a biological instrument for the removal of dead and infected human body tissues,
4) as a biological immunizing agent protecting young regenerating skin,
5) as a biological immunizing agent protecting young skin tissue that has not succeeded to establish immunity after regeneration against a repeated attack from the disease.

The treatment period for certain infectious skin diseases using live "Penicillium notatum" fungus lasts from three to five days.

In the future, it may be possible to use live "Penicillium notatum" fungus for augmenting the immunity lost by people under various specific circumstances: specific conditions of work and day-to-day activity, long-time presence in space, loss of or removal from constant gravitational weight, impaired individual immunity caused by space-related factors, and the emergence of new diseases.

It can supplement or replace the immunizing microflora of human internal organs.

It is likely that the fungus will be domesticated and new species thereof will be bred artificially and specifically for each organ of the human body.

It is essential that mankind learns how to use the live "Penicillium notatum" fungus in the field of medicine in view of the fact that its resilience exceeds the resilience of all life forms known to mankind, including the resilience of the microorganisms of the immunizing human microflora.

## Description

Method for treating infectious skin diseases using live "Penicillium notatum" fungus. Technical field - clinical medicine, section - dermatology.

### Prior art.

Similar methods for treating infectious skin diseases:
1 application of bactericidal ointments,
2 injection of antibiotics,
3 excision of tissues affected by pathogenic microorganisms by surgical operations (sometimes amputation of limbs),
4 method for treating festering wounds using blue blowfly larvae "Calliphora vomitoria", patent RU2177784 RU2180574.

All these methods have disadvantages:
1 the treating method using bactericidal ointments has a large time period and a small healing effect, bactericidal ointments are lost from the surface of skin parts of areas affected by pathogenic microorganisms, penetration into the infected tissue is not uniform and not deep, healing properties are quickly lost, chemical reactions with the environment occur;
2 injection of antibiotics into the human organism suppresses, and sometimes kills, some types of microorganisms of the own human immune microflora, poisons all of his organs, weakens the entire immunity of the ailing man;
3 surgical operations have very expensive and lengthy periods of treatment and lead to irrevocable casualties to the patient's health;
4 treating festering wounds using live blue blowfly larvae has a disadvantage related to the activities of the larvae in the wound, and a long period of treatment.

An analogue (prototype) of my invention is a method for treating festering wounds using blue blowfly larvae "Calliphora vomitoria", patent RU2177784 RU2180574.

Coincident features: blue blowfly larvae live on the surface of festering wounds, they produce a toxic, kill pathogenic microorganisms in festering human wounds, support man's immunity, struggle with pathogenic microorganisms from the depths of the human body.

The method for treating infectious skin diseases and festering wounds by using blue blowfly larvae "Calliphora vomitoria" is little effective, uncomfortable, toxics have an insufficient penetration depth into the infected areas of parts of the human skin, is very lengthy or time consuming.

### Disclosure of the invention.

### Method for treating with live "Penicillium notatum" fungus.

The population of the live "Penicillium notatum" fungus is previously grown on the basis of fine grain, put on areas or parts of the human body infected with pathogenic microorganisms, the entire infected area or part is covered by the fungal population, and it is held on the surface of the infected area or part until full recovery with the bread slice carrying the fungal population being replaced daily. Attended bread slices carrying a population of living "Penicillium notatum" fungus are fastened on the treated area or part of the body by bandages, leucoplasts and atop bandages protecting the polyethylene film from drying. During ligation the infected area or part is washed with warm clean water.

### Implementation of the invention.

The population of the live "Penicillium notatum" fungus is planted on the areas or parts of the human body infected with pathogenic microorganisms. The live "Penicillium notatum" fungus produces an antibiotic which is most effective against (this) kind of pathogenic microorganisms which are currently towards the "Penicillium notatum" fungus, delivers an antibiotic individually to each pathogenic microorganism, penetrates evenly, deeply and quickly into tissues of the human body unsustainably infected with pathogenic microorganisms, inhibits and kills pathogenic microorganisms, breaks down and removes tissues of the human body having lost vitality and being affected by pathogenic microorganisms, does not go to healthy tissues of the human body, protects against infection regenerated new skin on the surface of cured human tissues, lives in the upper layer of the regenerated skin until restoration of the own immune microflora on the healed area or part of the human skin, gives the skin a darker shade of green and protects the skin from the recurrence of the disease, leaves on the skin a cured part of the human body after restoration of the immune microflora of this part, leaves behind the own natural colour of the human skin, the former prior to the disease.

## Claims

1. Method for treating infectious skin diseases using live "Penicillium notatum" fungus, characterized as the analogue (prototype) - method for treating festering wounds using blue blowfly larvae in the role of a microorganism disinfecting the wound (Patent RU2177784 RU2180574) - by settling live "Penicillium notatum" fungus on parts or areas of the human body affected by pathogenic microorganisms,
**characterized by**:
settlement of the live "Penicillium notatum" fungus on parts or areas of the human body affected by pathogenic microorganisms:
the role of a nano-bio-surgical tool, that quickly and painlessly penetrates, subtly chooses, breaks down and removes from its thickness formations of the body affected by infection that lost their viability and tissues infected with pathogenic microorganisms, leaves intact and protects viable ones necessary for the regeneration of tissue residues,
the role of immunity, penetrates continuously, uniformly, intensely into the depth of tissues of the human body locally affected by pathogenic microorganisms and delivers them an antibiotic produced for (this) kind of pathogenic microorganisms individually for each pathogenic microorganism,
the role of a protective immunizing agent during a period of a regeneration process of soft subcutaneous tissues and tissues of the human body in the focus of the disease,
the role of immunizing for protection against recurrence of the disease during the period of recovery of the own immunity of the skin of the given body area or part,
ease of use,
reduction of the treating period of infectious skin diseases by the tens and hundreds of times,
reduction of economic costs for treatments of infectious skin diseases by the thousands of times,
avoiding surgical operations to amputate parts of the human body associated with the inability to cure the given disease with other medication methods.
